# EUROPÄISCHE PATENTSCHRIFT

(11) **EP 0 730 485 B1**
(45) Veröffentlichungstag und Bekanntmachung des Hinweises auf die Patenterteilung: **02.02.2000**
(21) Anmeldenummer: 95900741.0
(22) Anmeldetag: 15.11.1994
(51) Int. Cl.: B01D 3/34, B01D 1/18

(54) **VERFAHREN ZUR VEREINFACHTEN TRENNUNG VON MEHRSTOFFGEMISCHEN WENIGSTENS ANTEILIG ORGANISCHEN URSPRUNGS**
PROCESS FOR THE SIMPLIFIED SEPARATION OF COMPLEX MIXTURES AT LEAST PARTIALLY OF ORGANIC ORIGIN
PROCEDE DE SEPARATION SIMPLIFIEE DE MELANGES DE PLUSIEURS SUBSTANCES AU MOINS PARTIELLEMENT D'ORIGINE ORGANIQUE

(30) Priorität: 24.11.1993 DE 4340093
(43) Veröffentlichungstag der Anmeldung: 11.09.1996
(73) Patentinhaber: Henkel Kommanditgesellschaft auf Aktien, 40191 Düsseldorf (DE)
(72) Erfinder: RÄHSE, Wilfried, D-40589 Düsseldorf (DE); FUES, Johann-Friedrich, D-41516 Grevenbroich (DE); BÜTTGEN, Karl-Heinz, D-50170 Kerpen (DE); DICOI, Ovidiu, D-40789 Monheim (DE)
(86) Internationale Anmeldenummer: EP9403786
(87) Internationale Veröffentlichungsnummer: WO9514520

(56) Entgegenhaltungen:
- DE-A- 4 204 035
- DE-A- 4 206 050
- DE-C- 976 413
- FR-A- 910 829
- FR-A- 1 021 887
- FR-A- 2 253 542

## Beschreibung

### Gebiet der Erfindung

Die Erfindung beschreibt Vorschläge zur Intensivierung und/oder Vereinfachung der Trennung von Mehrstoffgemischen, die wenigstens anteilsweise organischen Ursprungs sind, unter Verwendung eines in der Gasphase eingesetzten Trägerstromes zum erleichterten Austrag der leichter flüchtigen Anteile des Einsatzgutes. Das Prinzip des erfindungsgemäßen Verfahrens lehnt sich damit an Reinigungsschritte an, die sich dem Fachbegriff des "Dämpfens mit Wasserdampf" unterordnen. Das erfindungsgemäße Arbeitsprinzip unterscheidet sich hiervon aber durch die Auswahl des Trägergasstromes, der in seiner Beschaffenheit und bezüglich der zum Einsatz kommenden Betriebsbedingungen im nachfolgenden im einzelnen geschildert ist und dabei nicht durch Wasserdampf sondern durch ein Trägergas wenigstens anteilig organischen Ursprungs gebildet ist. Gleichwohl können in weitem Umfang die dem Fachmann aus der Wasserdampfdestillation bekannten Maßnahmen zum Einsatz kommen.

### Stand der Technik

Die Arbeitsprinzipien der Wasserdampfdestillation zur Auftrennung von Stoffgemischen und insbesondere zur Reinigung von Wertstoffen oder Wertstoffgemischen wenigstens anteilsweise organischen Ursprungs sind altes chemisches Fachwissen; verwiesen sei beispielsweise auf L. Gattermann "Die Praxis des organischen Chemikers" 33. Auflage (1948), Walter De Gruyter & Co. Verlag, S. 26-28 und 252. Die hier für die Laborpraxis beschriebenen Gesetzmäßigkeiten werden in derart vielgestaltiger Weise in unterschiedlichsten technischen Arbeitsgebieten eingesetzt, daß hier nur auszugsweise auf eine Reihe charakteristischer Anwendungsfälle verwiesen werden kann.

Die Reinigung von Fetten und Ölen pflanzlichen oder tierischen Ursprungs umfaßt eine mehrstufige Behandlung, die gewöhnlich als letzten Verfahrensschritt die Dämpfung der vorgereinigten Ware vorsieht. Ein wesentliches technisches Ziel dieser Arbeitsstufe ist die Desodorierung des vorgereinigten Materials. Unerwünschte und insbesondere geruchlich störende und häufig nur in Spurenmengen vorliegende Begleitstoffe werden hier auf dem Wege der Wasserdampfdestillation vom Wertstoff beziehungsweise Wertstoffgemisch abgetrieben. Diese Stufe der Dämpfung kann aber auch schon als Destillationshilfe, beispielsweise zur erleichterten Abtrennung kurzkettiger Fettsäuren aus den natürlichen Fetten und Ölen eingesetzt werden. Zur einschlägigen Literatur sei beispielsweise verwiesen auf "Ullmanns Encyklopädie der technischen Chemie", 4. Auflage, Band 11 (1976), S. 479-486; Kirk-Othmer "Encyclopedia of Chemical Technology", 3. Ausgabe, Vol. 9 (1980), S. 816-820 sowie E. Bemardini "Vegetable Oils and Fats Processing" in "Oilseeds, Oils and Fats", Vol. II (1983), InterstampaRome, Kapitel VII, S. 221-251 (Deodorization of Fats and Oils). Die hier beschriebenen großtechnisch durchgeführten Reinigungsverfahren unter Benutzung des Prinzips der Wasserdampfdestillation beziehungsweise des Dämpfens arbeiten im Vakuum und bei hohen Temperaturen. Gedämpft wird beispielsweise im Druckbereich von 2 bis 30 mbar und Temperaturen von 150 bis 290°C. Die Dampfmenge und Behandlungsdauer hängen vom jeweils gewählten Verfahrenstyp ab. Bekannt sind absatzweise arbeitende, halbkontinuierlich geführte und kontinuierliche Verfahren. In allen Verfahrenstypen ist vorgesehen, den Wasserdampf feindispers durch das aufgeschmolzene und hocherhitzte Fett beziehungsweise Öl zu leiten, wobei beim Arbeiten in halbkontinuierlichen und kontinuierlichen Verfahrenstypen auch weitere Hilfen zur Vergrößerung der Oberfläche zwischen dem Wasserdampf und der zu reinigenden Ölphase vorgesehen sein können. Beschrieben ist dabei insbesondere der Einsatz von Kolonnen, die auch mit Füllkörpern, Packungen und dergleichen ausgerüstet sein können. Das zu reinigende Flüssiggut wird im Kolonnenbereich mit gespreiteter und damit vergrößerter Oberfläche dem durchstreichenden Wasserdampf ausgesetzt.

Die DE-C-976413 beschreibt ein Verfahren zum Herstellen von Estern in einem kontinuierlichen Verfahren, bei dem ein Gemisch aus einem Alkohol, einer verhältnismäßig wenig flüchtigen Carbonsäure oder einem Carbonsäureanhydrid oder einem sauren oder neutralen Ester aus einem verhältnismäßig flüchtigen Alkohol und einer Carbonsäure sowie einem sauren Katalysator im Gleichstrom von oben nach unten durch eine Reihe von erhitzten Reaktionszonen hindurchgeführt wird, wobei in das Veresterungsgemisch im Gegenstrom von unten nach oben ein Schleppmittel zum Entfernen des Reaktionswassers aus dem Reaktionsgemisch eingeführt wird. Das Veresterungsgemisch wird dabei durch eine Reihe von mindestens 3 und zweckmäßig höchstens 10 Reaktionszonen geführt, die mittels voneinander unabhängiger Heizvorrichtungen auf einer solchen Temperatur gehalten werden, daß eine Fraktionierung der Reaktionsteilnehmer weitgehend vermieden wird. Das Veresterungsgemisch liegt in den jeweiligen Reaktionszonen als geschlossene Phase vor und wird von dem dampfförmigen Schleppmittel durchströmt. Dabei können als Schleppmittel aromatische Kohlenwasserstoffe mit 6 bis 8 Kohlenstoffatomen verwendet werden, es ist aber auch möglich als Schleppmittel einen 1-wertigen Alkohol mit 1 bis 4 Kohlenstoffatomen einzusetzen, der gleichzeitig als Veresterungskomponente dient. Die einzelnen Reaktionszonen arbeiten nicht nach dem Prinzip der Sprühzone.

Die DE-A-4206050 beschreibt unter anderem ein Verfahren zur Herstellung von Aniontensidkonzentraten in Pulver- und/oder Granulatform, bei dem man wäßrige Lösungen, Emulsionen und/oder Suspensionen der Aniontenside beziehungsweise Tensidgemische und gegebenenfalls mitverwendeten Hilfs- und/oder Wertstoffe den Bedingungen der Trocknung in einer Sprühzone und/oder in Wirbelschicht unterwirft. Als die Sprühzone durchströmende Gasphase wird überhitzter Wasserdampf eingesetzt.

Der Einsatz einer überhitzten organischen Gasphase zur Reinigung eines vergleichsweise schwerer flüchtigen Einsatzmaterials wird in der FR-A-2253542 vorgeschlagen. Organische Lösungsmittel der unterschiedlichsten Art mit Siedepunkten im Bereich zwischen etwa 60 und 190°C unter Normalbedingungen sollen die überhitzte Gasphase ausbilden. Im einzelnen sind hier neben aliphatischen und alizyklischen gesättigten Kohlenwasserstoffverbindungen, aromatischen Kohlenwasserstoffen, aliphatischen linearen oder zyklischen Ethern, Ketonen, Phenol und Phenolethern auch aliphatische Alkohole, beispielsweise Ethanol, genannt. In der konkreten Ausführungsform soll dabei die überhitzte Gasphase zu dem unter Arbeitsbedingungen fließfähigen zu reinigenden Ausgangsgemisch in einem Fallfilmverdampfer im Gegenstrom geführt werden.

Ein anderes typisches Arbeitsgebiet für den Einsatz der Reinigung durch Dämpfen mit großtechnischer Bedeutung liegt in der Entfernung von Reststoffen auf Basis von Ethylenoxid und/oder Propylenoxid aus Reaktionsprodukten, die durch Ethoxylierung und/oder Propoxylierung organischer Verbindungen mit mindestens einem aktiven Wasserstoffatom hergestellt worden sind. Verbindungen dieser Art haben beispielsweise große Bedeutung als nichtionische Tenside oder als Zwischenprodukte für die Herstellung anionischer Tensidverbindungen. Ihre Anwendung erfolgt beispielsweise auf dem Gebiet der Wasch- und Reinigungsmittel, in großem Umfange aber auch auf dem Gebiet der Kosmetika oder der pharmazeutischen Hilfsstoffe. Die primär anfallenden Umsetzungsprodukte enthalten herstellungsbedingt Spuren von Ethylenoxid und/oder Propylenoxid sowie von unerwünschten Reaktionsfolgeprodukten wie Dioxan. Die Entfernung dieser Reststoffe aus den alkoxylierten Derivaten ist durch gesetzliche Regelungen vorgeschrieben und zwingender Schrift im Herstellungsverfahren. Die Wasserdampfdestillation beziehungsweise Dämpfung der primär anfallenden Reaktionsprodukte zur Entfernung der unerwünschten Verunreinigungen ist hier der in der Praxis großtechnisch eingesetzte Verfahrensschritt. Verwiesen sei in diesem Zusammenhang beispielsweise auf EP-A1-O 283 862, DE-A1-34 47 867, US-A-4 143 072 und die darin referierte Literatur.

Nach einer Modifizierung des genannten Arbeitsprinzips kann aber auch so vorgegangen werden, daß zu reinigende Wertstoffe beziehungsweise Wertstoffgemische vorzugsweise im Vakuum erhitzt, dabei eingedampft und über das Prinzip der Wasserdampfdestillation gereinigt werden. Die Mitverwendung von zusätzlichem gegebenenfalls überhitztem Wasserdampf ist dabei Stand der Technik. Die angefallenen wasserärmeren aber von Verunreinigungen befreiten Verfahrensprodukte können gewünschtenfalls nachfolgend wieder in wäßrige Zubereitungsform überführt werden. Verwiesen wird in diesem Zusammenhang beispielsweise auf DE-A1-30 44 488, in der ein Verfahren zur Herstellung von Ethersulfaten mit erniedrigtem Dioxangehalt beschrieben ist.

Bekannt ist weiterhin die Desodorierung und/oder Entfernung von unerwünschten Begleitstoffen unter Einsatz nicht kondensierbarer Gasphasen als Destillationshilfe durchzuführen. Das bevorzugte Hilfsmittel ist hier gasförmiger Stickstoff, der zum erleichterten Übertreiben der höherflüchtigen Anteile aus Stoffmischungen eingesetzt werden kann. Verwiesen wird in diesem Zusammenhang beispielsweise auf die JP-A-5414/81. Hier werden Polyalkylenglykolderivate - zum Beispiel zur Verwendung als Emulgatoren, Schmieröle, Ausgangsmaterialien für Kunststoffe, Waschmittel, Kosmetika und dergleichen - dadurch der Reinigung und Desodorierung unterworfen, daß in das zu reinigende fließfähige Material bei etwa 30 Torr und 90 bis 100°C gasförmiger Stickstoff oder Wasserdampf eingeblasen werden. Auch für das eingangs genannte Arbeitsgebiet der Deodorisierung von eßbaren Ölen und/oder Fetten wird in jüngster Zeit vorgeschlagen, anstelle von Wasserdampf nicht kondensierbare Inertgase, insbesondere Stickstoff als Stripping-Hilfe einzusetzen, siehe hierzu beispielsweise EP-A2-015 739.

Die US-A-4 443 634 beschreibt ein Verfahren zur Reinigung von Fettalkoholpolyglykolethern, bei dem das zu reinigende Gut in eine Kammer gesprüht wird, aus der die Verunreinigungen dampfförmig abgezogen werden. Als zu reinigendes Einsatzmaterial werden entsprechende Stoffmischungen mit weniger als 2 Gew.-% der abzutrennenden Verunreinigungen - bezogen auf flüssiges Einsatzmaterial - beschrieben. Das zu reinigende Gut soll dabei in einer inerten Atmosphäre versprüht werden, wobei der Druck so zu wählen ist, daß Tröpfchen mit einem Teilchendurchmesser von 50 bis 1.000 mm entstehen. Diese Tröpfchen sollen im Sekundenbereich der Inertgasatmosphäre ausgesetzt sein und dann gesammelt werden. Als Inertgase sind Stickstoff, Helium und Argon genannt. Die durch diese Sprühbehandlung abzutrennenden Verunreinigungen sind insbesondere Ethylenoxid, Propylenoxid, Dioxan, Wasser und Alkohol. Das Versprühen der Flüssigphase in den mit Inertgas erfüllten Raum kann auch mehrfach in aufeinanderfolgenden Verfahrensstufen wiederholt werden.

Die Anmelderin beschreibt in ihren älteren deutschen Patentanmeldungen DE-A-42 37 934 und DE-A-43 07 115 wichtige Verbesserungen zur Reinigung organischer Einsatzmaterialien mit überhitztem Wasserdampf. Das zuerst genannte Schutzrecht schildert ein Verfahren zur Verbesserung des Reinheitsgrades und insbesondere der Beschaffenheit von Farbe und Geruch in Wertstoffen und Wertstoffgemischen aus dem Bereich der Netz-, Wasch-und/oder Reinigungsmittel (Einsatzgut), das dadurch gekennzeichnet ist, daß man ein mit Verunreinigungen belastetes Einsatzgut mit überhitztem Wasserdampf behandelt, wobei zur Ausbildung von Farbverbesserungen Bleichmittel im Einsatzgut mitverwendet werden können. Bevorzugt wird dabei das belastete Einsatzgut in feinteiliger Form und insbesondere in Abmischung mit Wasser der Behandlung mit überhitztem Wasserdampf unterworfen und gewünschtenfalls dabei wenigstens anteilsweise aufgetrocknet. Diese Behandlung mit dem überhitzten Wasserdampf erfolgt zweckmäßigerweise in einer Sprühzone und/oder einer Wirbelschicht.

Die zweite der zuvor genannten älteren Anmeldungen betrifft ein Verfahren zur Intensivierung und/oder Beschleunigung der destillativen Trennung von Mehrstoffgemischen, wenigstens anteilsweise organischen Ursprungs, unter Einsatz eines Wasserdampfstromes zum erleichterten Austrag wasserdampfflüchtiger Anteile des Einsatzgutes (Dämpfen), das dadurch gekennzeichnet ist, daß man ein unter den Behandlungsbedingungen fließfähiges Einsatzgut in feinteilig versprühter Form dämpft. Dieses Dämpfen wird vorzugsweise mit bei Arbeitsdruck überhitztem Wasserdampf durchgeführt. In einer besonders wichtigen Ausführungsform erfolgt das Versprühen der zu reinigenden Flüssigphase unter Mithilfe eines Treibgases, wobei das Arbeiten und der Einsatz von Mehrstoff-Sprühdüsen besonders vorteilhaft sein kann. Als Treibgas wird in den wichtigsten Ausführungsformen dieser technischen Lehre wenigstens anteilsweise Wasserdampf und insbesondere überhitzter Wasserdampf eingesetzt.

Der Offenbarungsinhalt der beiden genannten älteren deutschen Patentanmeldungen DE-A-42 37 934 und DE-A-43 07 115 wird hiermit ausdrücklich auch zum Gegenstand der vorliegenden Erfindungsoffenbarung gemacht. Die in diesen Anmeldungen geschilderten Prinzipien haben Gültigkeit auch für die Lehre der im nachfolgenden geschilderten Erfindung, wobei die erfindungsgemäße Abwandlung jetzt darin liegt, als gasförmiges Schleppmittel anstelle des überhitzten Wasserdampfes eine ausgewählte überhitzte Dampfphase wenigstens anteilsweise organischen Ursprungs einzusetzen.

Bevor auf die erfindungsgemäße Lehre in ihren Einzelheiten eingegangen wird, sei noch auf ein ganz anderes Gebiet des großtechnischen Einsatzes zur Mithilfe der Prinzipien der Wasserdampfdestillation verwiesen: Bekannt ist, daß ganz allgemein schwierige destillative Trennungen durch Mitverwendung des Arbeitsprinzips der Dampfdestillation erleichtert werden können. So ist beispielsweise die Entfernung nicht umgesetzter Fettalkoholanteile im Rahmen der Herstellung von niotensidischen Komponenten aus der Klasse der Alkylpolyglykoside (APG), beschrieben in EP-B-0 092 876 und weiterführenden entsprechenden Anmeldungen. Hier wird die Destillation des APG-haltigen Rohproduktes im Dünnschichtverdampfer unter Vakuum durchgeführt. Der Stoffaustrag des abzudestillierenden freien Fettalkohols kann dadurch gefördert werden, daß das breitflächig auf der Innenfläche des Dünnschichtverdampfers verstrichene Einsatzgut mit seiner vergrößerten Oberfläche dem durchstreichenden Wasserdampfstrom ausgesetzt wird.

### Die erfindungsgemäße Lehre

Gegenstand der Erfindung ist demgegenüber in einer ersten Ausführungsform ein Verfahren zur erleichterten Trennung eines bei Arbeitstemperaturen fließfähigen, organische Anteile enthaltenden Mehrstoffgemisches - im nachfolgenden auch als "Einsatzmaterial" bezeichnet - durch feinteiliges Versprühen des Einsatzmaterials in eine Sprühzone, die von einem gasförmigen Schleppmittel durchströmt wird, das der Sprühzone - bezogen auf deren Arbeitsbedingungen - als überhitzte Dampfphase zugeführt und mit Anteilen des Einsatzmaterials beladen wieder abgetrennt wird. Gekennzeichnet ist die erfindungsgemäße Lehre dadurch, daß monofunktionelle Alkohole mit 1 bis 3 C-Atomen oder deren Abmischungen mit Wasser als überhitzte Dampfphase und damit als gasförmiges Schleppmittel eingesetzt werden. Im Rahmen der Erfindungsbeschreibung wird diese als Schleppmittel eingesetzte überhitzte Dampfphase auch als "super-heated carrier fluid/SHCF" bezeichnet.

Die erfindungsgemäße Lehre nutzt die anhand der Wasserdampfdestillation dargestellten bekannten Verfahrenserleichterungen zur Stofftrennung. Erfindungsgemäß ist jetzt aber vorgesehen, als Schleppmittel - im Sinne der erfindungsgemäßen Definition "SHCF" - niedere monofunktionelle Alkohole oder deren Abmischungen mit Wasser im Zustand der überhitzten Dampf- beziehungsweise Gasphase - bezogen jeweils auf die Arbeitsbedingungen der Trennstufe - einzusetzen. Bevorzugte Alkohole für die Ausbildung der SHCF sind im Sinne der erfindungsgemäßen Lehre primäre Alkanole mit 1 bis 3 C-Atomen. Besondere Bedeutung kann dem Einsatz von Methanol und/oder Ethanol als SHCF zukommen.

Bevorzugte Arbeitsmittel für den Einsatz als SHCF können die genannten niederen monofunktionellen Alkohole als solche sein. Wie angegeben erfaßt die erfindungsgemäße Lehre darüberhinaus aber auch die Abmischungen dieser Alkohole mit Wasser, wobei in wichtigen Ausführungsformen der Alkoholanteil überwiegt. Aber auch der Einsatz von Alkohol/Wasser-Gemischen mit überwiegendem Wasseranteil fällt in den Rahmen des erfindungsgemäßen Handelns, wobei Einzelheiten hierzu nachfolgend noch angegeben werden.

Erfindungsgemäß wird mit fließfähigen, unter Arbeitsbedingungen flüssigen Zubereitungen des aufzuarbeitenden Mehrstoffgemisches gearbeitet und das zu trennende Mehrstoffgemisch der gasförmigen überhitzten Alkohol-/ beziehungsweise Alkohol/Wasser-Phase mit vergrößerter Oberfläche ausgesetzt. Das erfolgt durch Versprühen der zu reinigenden fließfähigen Flüssigphase in einer Sprühzone, wobei insbesondere auch hier unter Mithilfe eines Treibgases gearbeitet werden kann. Als Treibgas wird in den wichtigsten Ausführungsformen der Erfindung wenigstens anteilsweise die alkoholbasierte SHCF-Phase im Sinne der erfindungsgemäßen Definition eingesetzt.

Die Erfindung betrifft in weiterführenden Ausführungsformen die Anwendung dieses Verfahrens in verschiedenartigsten technologischen Teilgebieten. In der überwiegenden Zahl dieser Anwendungsfälle kommt die SHCF-Phase als Destillationshilfe bei der Auftrennung von wenigstens anteilsweise schwer flüchtigen Stoffmischungen organischen Ursprungs zum Einsatz. So kann dieses Arbeitsmittel insbesondere zur Verbesserung des Reinheitsgrades von Wertstoffen und Wertstoffgemischen pflanzlichen und/oder synthetischen Ursprungs eingesetzt werden, insbesondere zur Reinigung von Fetten und/oder Ölen, zum Beispiel für deren Einsatz als Nahrungsmittel, von Komponenten aus dem Bereich der Kosmetika, der Netz-, Wasch- und/oder Reinigungsmittel sowie der pharmazeutischen Hilfsstoffe.

Besondere Bedeutung kommt der Anwendung des erfindungsgemäßen Verfahrensprinzip in einer Ausgestaltung der erleichterten Reinigung der Reaktionsprodukte aus der Umesterung von Glyceridestern, insbesondere Triglyceriden natürlichen Ursprungs, mit monofunktionellen Alkanolen mit insbesondere 1 bis 5 C-Atomen zu. Hier kann insbesondere die Verwendung von überhitztem Methanol und/oder Ethanol als SHCF-Strom im Sinne der erfindungsgemäßen Lehre wichtige Verfahrensvereinfachungen bei der Reinigung der Methyl- und/oder Ethylester von Fettsäuren natürlichen Ursprungs liefern, die durch Umesterung mit Methanol und/oder Ethanol aus den als Naturstoffe anfallenden entsprechenden Glyceriden gewonnen werden.

Es leuchtet für das zuletzt genannte Arbeitsgebiet sofort ein: Niedere Alkanole und insbesondere Methanol und/oder Ethanol stehen hier als Reaktanten für die Umesterung in den heute in großtechnischem Umfang betriebenen Anlagen zur Verfügung. Dabei wird mit beträchtlichen Kreislaufströmen, gerade auch dieser niederen monofunktionellen Alkanole, gearbeitet. Die erfindungsgemäße Lehre integriert in dieser Ausführungsform in die bestehenden Verfahrensabläufe einen Reinigungsschritt, in dem die SHCF-Phase auf Basis von beispielsweise Methanol zur wirkungsvollen Auftrennung des Rohproduktes oder eines nicht hinreichend gereinigten Produkts aus der Umesterungsstufe Verwendung findet. Durch ein solches "Dämpfen mit überhitztem Methanol" können in technisch extrem vereinfachter Weise unter optimaler Schonung des zu reinigenden Gutes hochwertige Trennergebnisse erhalten werden. Die zu dieser Trennstufe eingesetzten Arbeitsmittel und Energie(teil)beträge gehen nicht verloren, sondern können in das Gesamtverfahren reintegriert werden. Es erschließen sich damit wirtschaftliche und technologische Möglichkeiten und Verbesserungen, wie sie bisher als nicht möglich angesehen worden sind.

Die erfindungsgemäße Lehre erschließt damit in einer wichtigen Ausführungsform den technologisch und wirtschaftlich verbesserten Zugang zu naturstoffbasierten Großprodukten, wie sie beispielsweise auf dem Gebiet der Verbrennungskraftstoffe heute als sogenannter Biodiesel gewünscht werden. Die hinreichende Reinigung entsprechender Fettsäure-Methylestergemische und insbesondere die hinreichende Abtrennung von Glycerin aus den Primärprodukten der Umesterung wird bei Einsatz des erfindungsgemäßen Arbeitsprinzips in bisher nicht zugänglicher Weise erleichtert.

### Einzelheiten zur erfindungsgemäßen Lehre

Ein wesentlicher Kern des erfindungsgemäßen Handelns liegt in dem Austausch des bisher als Schleppmittel eingesetzten überhitzten Wasserdampfs durch die jetzt organisch basierte dampfförmige Schleppmittelphase. Die niederen monofunktionellen Alkohole oder deren Abmischungen mit Wasser werden unter Arbeitsbedingungen eingesetzt, daß sie - bezogen auf die jeweils konkret gewählten Arbeitsparameter - in der Trennstufe in überhitzter Dampfphase vorliegen. Diese alkoholbasierte Dampfphase übernimmt die Funktion, die in dem referierten Wissen zur erleichterten Stofftrennung unter Einsatz von überhitztem Wasserdampf diesem Arbeitsmittel zukommt und insbesondere in der erleichterten Abtrennung der leichter flüchtigen Anteile vom Einsatzgemisch liegt.

Bevorzugte Schleppmittel zur Ausbildung des SHCF sind niedere monofunktionelle primäre Alkanole mit 1 bis 3 C-Atomen. Besondere Bedeutung kommt in diesem Zusammenhang dem Methanol und/oder dem Ethanol zu, deren Siedepunkte unter Normaldruck bekanntlich bei ca. 65°C, beziehungsweise ca. 78°C, liegen. Auch n-Propanol und Isopropylalkohol (sec. Alkanol) mit ihren unter Normaldruck ebenfalls noch unterhalb 100°C liegenden Siedepunkten bringen in der praktischen Verwirklichung des neuen Verfahrens Arbeitsbedingungen, die sich - bezogen auf die Siedecharakteristika des Schleppmittels - dem Dämpfen mit reinem Wasserdampf stark annähern.

Die niederen monofunktionellen Alkohole mit 1 bis 3 C-Atomen können als individuelle Reinsubstanzen in der SHCF zum Einsatz kommen. Es können aber auch Abmischungen von 2 oder mehr Alkoholen der angegebenen Art als SHCF Verwendung finden. So kann beispielsweise das erfindungsgemäße Verfahren mit Methanol als Schleppmittelphase ebenso durchgeführt werden wie mit Gemischen aus beispielsweise Methanol und Ethanol oder niederen Alkanolen mit 1 bis 3 C-Atomen. Unabhängig hiervon umfaßt die Erfindung die Möglichkeit Wasser als zusätzliche Mischungskomponente im Rahmen des im überhitzten Zustand eingesetzten Schleppmittels einzusetzen. Auch hier können Abmischungen von Wasser mit individuellen monofunktionellen niederen Alkoholen oder Abmischungen von Wasser mit Alkoholgemischen vorliegen.

Im einzelnen gilt dabei daß wenigstens ein substantieller Anteil der SHCF-Phase durch einen oder mehrere der niederen Alkohole gebildet ist. So liegt vorzugsweise der Gehalt an niederen monofunktionellen Alkoholen im SHCF bei wenigstens etwa 10 bis 25 Gew.-%. Höhere Werte von beispielsweise wenigstens etwa 30 oder wenigstens etwa 40 Gew.-% sind bevorzugt. In den wichtigsten Ausführungsformen macht der niedere mononfunktionelle Alkohol - beziehungsweise die entsprechende Alkoholabmischung - wenigstens etwa 50 Gew.-% der SHCF-Phase aus. Vergleichsweise wasserarme Mischphasen können besonders bevorzugte Ausführungsformen der Erfindung darstellen. Hier liegt der Alkoholgehalt in der SHCF-Phase bei wenigstens etwa 70 Gew.-% und insbesondere bei wenigstens etwa 80 bis 85 Gew.-%, vorzugsweise bei oder über 90 Gew.-% - Gew.-% in allen Fällen bezogen auf die SHCF-Phase.

Die Mitverwendung von Wasser in der als Schleppmittel eingesetzten SHCF-Phase auf Basis der niederen Alkohole kann insbesondere dann Bedeutung bekommen, wenn das erfindungsgemäße Verfahren auf wasserhaltige Einsatzmaterialien angewendet und dabei gleichzeitig eine Trocknung dieser Einsatzmaterialien mit der erfindungsgemäßen Behandlung verbunden ist. Hierbei wird Wasser als dampfförmiger Bestandteil der vom Einsatzgut abzutrennenden Gasphase Anteil der gegebenenfalls im Kreislauf zu führenden SHCF-Phase. Die Möglichkeit einen Anteil dieses Wassers in der gegebenenfalls im Kreislauf geführten überhitzten Dampfphase zu belassen kann zu Verfahrensvereinfachungen, gegebenenfalls auch zu Intensivierungen des erfindungsgemäßen Reinigungsverfahrens führen. Besondere Bedeutung kann in diesem Zusammenhang der Verwendung von Wasser/Alkohol-Gemischen zukommen, die etwa azeotropen Mischungsverhältnissen hier betroffener Wasser/Alkohol-Gemische entsprechen. Im einzelnen kann hier auf das allgemeine chemische Fachwissen zu diesen Alkohol/Wasser-Gemischen verwiesen werden.

Die als SHCF erfindungsgemäß eingesetzten Alkohole oder Alkohol/WasserGemische werden bevorzugt mit Einsatztemperaturen in die Trennstufe eingebracht, die wenigstens leicht oberhalb des Siedepunktes der höchstsiedenden Komponente des jeweils verwendeten SHCF bei den Arbeitsbedingungen der Trennstufe liegen. Es kann dementsprechend zweckmäßig sein, die Einsatztemperatur des überhitzten SHCF-Stromes wenigstens etwa 5 bis 10°C, insbesondere wenigstens etwa 20°C und zweckmäßigerweise wenigstens etwa 50°C oberhalb der betroffenen Siedetemperatur bei Arbeitsbedingungen in die Trennstufe einzuführen. Einsatztemperaturen des jeweiligen SHCFStromes im Bereich von wenigstens 150 bis 200°C oberhalb dieser Siedetemperatur beim Arbeitsdruck der Reinigungsstufe sind häufig bevorzugte Arbeitsparameter. Im einzelnen sind hier eine Mehrzahl von Parametern im Sinne allgemeinen technischen Fachwissens zu berücksichtigen, wobei beispielhaft aufgezählt seien: Die bestimmte Wahl der jeweils gewählten SHCF-Komponente(n), die Temperatursensitivität des zu behandelnden Mehrstoffgutes, das Massenverhältnis der SHCF-Phase zur Mehrstoffmischung, die Verweildauer des zu behandelnden Mehrkomponentengemisches in Gegenwart des überhitzten SHCF und dergleichen.

Durch geeignete Wahl der Arbeitsbedingungen und unter Berücksichtigung der dem Fachmann im jeweils konkreten Einzelfall bekannten oder leicht zu ermittelnden Parameter kann auch mit beträchtlichen absoluten Einsatztemperaturen des SHCF-Stromes gearbeitet werden. Diese - jetzt von der jeweiligen Siedetemperatur des Alkanols unter Arbeitsbedingungen losgelösten - Arbeitstemperaturen können beispielsweise im Bereich bis etwa 500°C und vorzugsweise im Bereich von etwa 80 bis 400°C liegen. Lediglich bei der Behandlung hoch-temperatursensitiver Einsatzmaterialien, die beispielsweise Mischungskomponenten mit Schädigungstemperaturen deutlich unter 100°C aufweisen, wird mit entsprechend abgesenkten Einsatztemperaturen des SHCF-Stromes gearbeitet werden.

Möglich wird eine solche Modifikation des Trennverfahrens im erfindungsgemäßen Sinne insbesondere auch durch die Steuerung des Arbeitsdruckes. Die aus der Stofftrennung mit Heißdampf dem Fachmann bekannten Parameter sind hier sinngemäß anzuwenden. So wird es - schon aus Gründen der Verfahrensvereinfachung - häufig wünschenswert sein, im Bereich des Normaldrucks oder nur schwach abgesenkter beziehungsweise schwach erhöhter Drucke zu arbeiten. Auch in großtechnischen Anlagen lassen sich mühelos Unterbeziehungsweise Überdrucke im Bereich bis beispielsweise jeweils etwa 150 mbar, vorzugsweise bis etwa 100 mbar, Abweichung von Normaldruck einstellen. Aber auch sehr viel stärkere Abweichungen des Arbeitsdruckes sind in der großtechnischen Praxis für vergleichbare Verfahren verwirklicht. So erfolgt das bekannte Deodorisieren natürlicher Fette und Öle durch Dämpfen gemäß den Angaben der zitierten einschlägigen Literatur bei stark erniedrigten Drucken, beispielsweise im Bereich von etwa 5 bis 20 mbar, gleichzeitig aber unter Einsatz von hohen Guttemperaturen, die deutlich über 100°C, zum Beispiel im Bereich von 150 bis 270°C, liegen können. In den Rahmen des erfindungsgemäßen Handelns fallen selbstverständlich gerade auch solche extremen Kombinationen der Arbeitsbedingungen von Druck und Temperatur, wobei die Temperatur des zum Einsatz kommenden überhitzten SHCF der Temperatur des zu dämpfenden Gutes etwa entsprechen kann, gewünschtenfalls allerdings auch darunter liegen kann. Im allgemeinen wird es jedoch eher bevorzugt sein eine SHCF Eintrittstemperatur zu wählen, die nochmal oberhalb der Einsatztemperatur des Flüssiggutes liegt. Allgemeines Fachwissen macht verständlich, daß durch die zuletzt genannte Ausführungsform, - die ja die Möglichkeit der Zuführung von zusätzlicher Verdampfungsenergie über den eingesetzten überhitzten SHCF-Strom ermöglicht - optimierte Verfahrensergebnisse zur Beschleunigung und/oder zur Intensivierung des auszutragenden Gutanteiles möglich werden.

Die Mindesttemperatur des zu behandelnden Einsatzmateriales in der Trennstufe ergibt sich aus den Arbeitsbedingungen dieser Trennstufe und dabei insbesondere aus den Parametern Arbeitsdruck und Siedetemperatur des SHCF beim jeweils vorgegebenen Arbeitsdruck. Wird das zu reinigende Einsatzmaterial der Trennstufe mit niedrigeren Temperaturen als der Siedetemperatur des SHCF unter Arbeitsbedingungen zugeführt, dann kondensiert zunächst SHCF auf dem Einsatzmaterial unter Abgabe der Kondensationswärme solange auf, bis die Siedetemperatur des SHCF unter Arbeitsbedingungen eingestellt ist. In Sonderfällen kann ein solches Vorgehen sinnvoll sein. Im allgemeinen wird eine solche vergleichsweise komplexere Reaktionsführung aber nicht gewünscht sein. Hier gilt dann das bevorzugte Verfahrenselement, daß das Einsatzmaterial mit einer Eigentemperatur der Trennstufe zugeführt wird, die wenigstens etwa der Siedetemperatur des als SHCF eingesetzten Alkanols beziehungsweise Alkanol/Wasser-Gemisches unter Arbeitsbedingungen entspricht. Bevorzugt liegt die Eigentemperatur des der Trennstufe zugeführten Einsatzmaterials über dieser Grenztemperatur. Dabei kann sie im vergleichsweise beschränkten Bereich darüberliegen, beispielsweise im Bereich bis zu 50 oder bis zu 100°C oberhalb der zuvor definierten Grenztemperatur. Hinreichende Schwerflüchtigkeit des zu reinigenden Einsatzmaterials in seinen Hauptkomponenten vorausgesetzt, können aber durchaus wesentlich höhere Einsatztemperaturen für das der Trennstufe zuzuführende Einsatzmaterial eingestellt werden. Im einzelnen gilt hier technologisches Fachwissen.

Erfindungsgemäß werden fließfähige Einsatzmaterialien dadurch dem Stoffaustausch mit vergrößerter Oberfläche angeboten, daß das Flüssiggut versprüht wird. Besonders geeignet ist in diesem Sinne der Einsatz von Sprühzonen, die von den erfindungsgemäßen SHCF-Phasen durchströmt werden. Im allgemeinen ist dabei weiterhin bevorzugt, die SHCF-Phase der Trennstufe kontinuierlich zuzuführen und daraus wieder abzunehmen, während das zu reinigende Einsatzmaterial absatzweise und/oder kontinuierlich der Trennstufe zugeführt werden kann.

In besonders wichtigen Ausführungsformen macht die erfindungsgemäße Lehre von den Prinzipien Gebrauch, die Gegenstand der zuvor benannten älteren Anmeldungen der Anmelderin gemäß DE-A-42 37 934 und DE-A-43 07 115 sind, und deren Arbeitsregeln bereits zum Gegenstand auch der vorliegenden Erfindungsoffenbarung gemacht worden sind. Zur Vervollständigung der Offenbarung seien hier kurz die wesentlichen Elemente, insbesondere des zuletzt genannten Schutzrechts, aufgezählt: Ein wesentlicher Kern auch des jetzt gegebenen erfindungsgemäßen Handelns liegt in der nachfolgenden Vertauschung: die konventionelle Desodorierung von beispielsweise Fetten und Ölen im batch-Verfahren legt die zu desodorierende Flüssigkeit in geschlossener Phase vor, der zum Dämpfen eingesetzte Wasserdampf wird über beispielsweise sternförmige oder ringförmige Injektionssysteme in einer Vielzahl von Austrittsöffnungen für den Wasserdampf fein-dispers in die zu reinigende geschlossene Flüssigphase ein- und durch sie hindurch geleitet. Die Lehre der Erfindung kehrt dieses Arbeitsprinzip um. Das unter den Behandlungsbedingungen fließfähige Einsatzgut wird in feinteilig versprühter Form mit dem Schleppmittel auf Basis des SHCF in Phasenkontakt gebracht. Das SHCF bildet dabei die geschlossene Phase. Das Ergebnis dieser Umkehr ist zunächst einmal eine beträchtliche Vergrößerung der für den Stoffübertritt aus der Flüssigphase in die Dampfphase entscheidenden Flüssigkeitsoberfläche pro Volumeneinheit der zu behandelnden Flüssigphase. Die spezifische und für den Stoffaustausch entscheidende Flüssigkeitsoberfläche kann beispielsweise um den Faktor 10² bis 10⁵ - im Mittel etwa um den Faktor 10³ - vergrößert werden.

Diese Anbietungsform der zu behandelnden Flüssigphase des Einsatzgutes mit substantiell vergrößerter Oberfläche schafft die Möglichkeit zur extremen Intensivierung und/oder Beschleunigung der Trennung der Mehrstoffgemische im Sinne der erfindungsgemäßen Zielsetzung. Es sind jetzt nicht mehr Behandlungszeiträume im Bereich einiger Stunden notwendig, vergleichsbare Stoffübergänge in die überhitzte SHCF-Phase können im Sekundenbereich erzielt werden. Die damit verbundenen Vorteile leuchten sofort ein: Es wird nicht nur ein sehr viel zügigeres Aufbereitungsverfahren möglich, insbesondere gelingt es erfindungsgemäß, die jeweilige Temperaturbelastung des zu reinigenden Gutes im vorbestimmten Rahmen sicher zu steuern. Das Versprühen des zu reinigenden Flüssiggutes kann in an sich bekannter Weise vorgenommen werden. Dabei steht das breite Angebot der einschlägigen Technik zu Einstoff- und/oder Mehrstoffdüsen und die damit verbundenen Verfahrenstechniken beziehungsweise Verfahrensparameter zur Verfügung. Die Aufrechterhaltung langer Verweilzeiten des zu reinigenden Gutes im versprühten Zustand ist nicht erforderlich. Die Überführung des zu reinigenden Einsatzmaterials in den feinteilig versprühten Zustand und seine Interaktion mit der geschlossenen SHCF-Phase kann gewünschtenfalls mehrfach wiederholt werden. Dabei kann in den einzelnen Sprühstufen mit dem bereits zuvor eingesetzten SHCF-Strom und/oder mit entsprechendem überhitzten Frischmaterial gearbeitet werden. Das mehrfache Versprühen kann in nur einer Arbeitseinheit oder in einer Mehrzahl voneinander getrennter und aufeinander folgender Arbeitseinheiten erfolgen. Selbst beim Arbeiten mit Mehrfach-Versprühung werden Belastungszeiträume für das zu behandelnde Einsatzmaterial im Minutenbereich nicht oder nur in Sonderfällen überschritten.

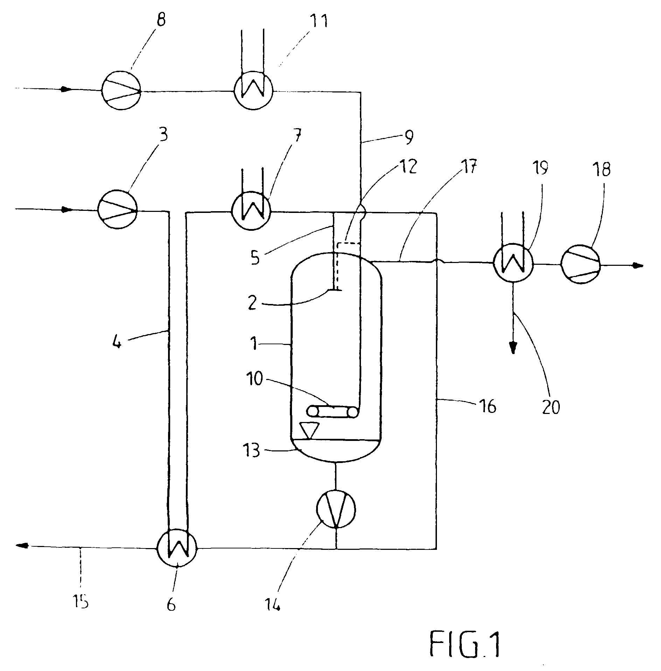

Auch die erfindungsgemäße Technologie erlaubt das absatzweise oder das kontinuierliche Verfahren. Beispiele für charakteristische Ausführungsformen der erfindungsgemäßen Verfahrensführung finden sich in den beigefügten Figuren 1 bis3, auf die nachfolgend noch im einzelnen eingegangen wird.

In einer besonders bevorzugten Ausgestaltung der Erfindung wird die zu reinigende fließfähige Phase unter Mithilfe eines Treibgases versprüht. Die einschlägige Technik kennt die verschiedenartigsten Ausgestaltungen solcher Sprühvorrichtungen, insbesondere Sprühdüsen. Verwiesen wird auf die einschlägige Fachliteratur, siehe hierzu beispielsweise H. Brauer "Grundlagen der Einphasen- und Mehrphasenströmungen" in GRUNDLAGEN DER CHEMISCHEN TECHNIK, Verfahrenstechnik der chemischen und verwandter Industrien, Verlag Sauerländer, Aarau und Frankfurt am Main (1971), S. 308-323, A.H. Lefebvre "Atomization and Sprays" Hemisphere Publishing Corp. New York (1989), S. 10-20, Chemical Engineering, Vol. 2, Unit Operations (2nd Edition - 1968) Pergamon Press, Oxford, New York, S. 602-617 sowie R.H. Perry et al. in "Chemical Engineering Handbook", (5th Edition - 1975), Mac Graw-Hill Book Co., New York "Phase Dispersion/Liquid-in-Gas Dispersions", S. 18-61 bis 18-65.

In erfindungsgemäß besonders bevorzugten Ausführungsformen wird unter Einsatz von Mehrstoffsprühdüsen und unter Mitverwendung von Treibgas gearbeitet, wobei in der wichtigsten Ausführungsform der Erfindung als Treibgas wenigstens anteilsweise SHCF-Phase im Sinne der erfindungsgemäßen Definition, d.h. also überhitzte niedere Alkohole oder entsprechende Alkohol/Wasser-Gemische zum Einsatz kommen. Im hier betroffenen Kern des erfindungsgemäßen Handelns ist die wichtigste Ausführungsform der Einsatz des überhitzten SHCF als ausschließliches Treibgas zum Versprühen des fließfähigen Mehrkomponentengemisches und dessen Überführung in die fein-disperse Flüssigphase. Es hat sich gezeigt, daß - wohl durch die intensive Vermischung im Sprühvorgang - beim Einsatz des erfindungsgemäß gewählten Treibgases der Stoffübergang der aus der Flüssigphase abzutrennenden Komponenten in die SHCF-Phase derart intensiviert wird, daß das im jeweiligen Verfahrenszyklus zu erreichende Reinigungsergebnis innerhalb von Sekundenbruchteilen eingestellt werden kann. Die Wirkungsweise und die Vorteile des erfindungsgemäßen Verfahrens werden hier einleuchtend verständlich. Unter Berücksichtigung der in der zitierten Literatur geschilderten jeweiligen Entstehungsgeschichte der versprühten Tropfen, die in der Regel eine lamellare Flüssigphasenspreitung bei extrem geringer Dichte der Flüssigphase einschließt, wird die Intensität der erfindungsgemäßen Maßnahme bezüglich der Einstellung des Reinigungsergebnisses verständlich. Das allgemeine Fachwissen des Verfahrenstechnikers zur Verstärkung dieses Effektes durch Auswahl geeigneter Mehrstoffdüsen kann darüberhinaus hier im Rahmen des erfindungsgemäßen Handelns eingesetzt werden.

Die Lehre der Erfindung ermöglicht in einer Ausgestaltung die Verwendung praktisch des insgesamt zum Einsatz kommenden SHCF als Treibmittel beim Zerstäubungsvorgang der zu reinigenden Flüssiggphase. In wichtigen Ausführungsformen wird allerdings zusätzlich vorgesehen, daß durch einen Teilstrom von überhitztem SHCF eine vorgegebene Strömungsrichtung der Gasphase im Sprühraum eingestellt und aufrechterhalten wird. So kann beispielsweise in aufrechtstehenden Reaktionsräumen derart im Gegenstrom gearbeitet werden, daß das Versprühen des flüssigen Gutes mit dem SHCF als Treibgas von oben nach unten gerichtet erfolgt, während gleichzeitig ein Teilstrom des überhitzten SHCF von unten dem versprühten Gut entgegengerichtet ist. Insgesamt sind aber hier praktisch beliebige Kombinationen von Gleichstrom und/oder Gegenstrom der Gas- und Flüssigphasen in an sich bekannter Weise einstellbar.

Die Mengenverhältnisse zwischen dem zu reinigenden Einsatzmaterial und dem als Schleppmittel eingesetzten SHCF werden durch das jeweils gewünschte beziehungsweise notwendige Ausmaß der Dämpfung des zu reinigenden Gutes mit dem überhitzten SHCF bestimmt. Grundsätzlich gelten hier die dem Fachmann vertrauten Mengenverhältnisse aus dem Dämpfen mit überhitztem Wasserdampf als Schleppmittel. So können auch im erfindungsgemäß modifizierten Reinigungsverfahren die jeweils als SHCF-Phase eingesetzten niederen Alkohole oder Alkohol/Wasser-Gemische, beispielsweise in Mengen von 0,5 bis 20 Gew.-%, vorzugsweise in Mengen von etwa 1 bis 15 Gew.-% -Gew.-% jeweils bezogen auf das eingesetzte und der Reinigung zu unterwerfende Einsatzmaterial - benötigt werden. In vielen Fällen praktischen Arbeitens liegen die Mengen des als überhitzte Dampfphase einzuführenden Alkohols beziehungsweise Alkoholgemisches im Bereich von etwa 1 bis 10 Gew.-% und insbesondere im Bereich von etwa 2 bis 5 Gew.-% - Gew.-% auch hier bezogen auf das Gewicht des zu reinigenden flüssigen und/oder festen Einsatzmaterials. Die hier angegebenen Zahlenwerte haben nicht nur Gültigkeit für den Einsatz von im wesentlichen wasserfreien Einsatzmaterialien, auch die im nachfolgenden geschilderte Variation des Verfahrens, die mit wasserhaltigen Einsatzmaterialien arbeitet und eine wenigstens anteilige Auftrocknung des Einsatzmaterials einschließt, wird häufig mit Mengen der SHCF-Phase im bisher angegebenen Bereich auskommen. Hier kann allerdings die Verwendung entsprechender Alkoholbeträge im oberen Bereich der angegebenen Zahlenwerte und insbesondere auch bei gegebenenfalls noch höheren Mengen an SHCF-Phase zweckmäßig und/oder notwendig sein. Im einzelnen gilt für die zuletzt angesprochene Ausführungsform der erfindungsgemäßen Lehre:

Die nahe Beziehung zwischen dem Dämpfen mit überhitztem Wasserdampf nach den Angaben des Standes der Technik einerseits und der erfindungsgemäßen Modifikation unter Einsatz des überhitzten SHCF-Stromes auf Basis niederer Alkohole führt dazu, daß auch im Sinne des erfindungsgemäßen Handelns das Sachgebiet der Trocknungsverfahren angesprochen ist, die mit überhitzten Gasphasen als Trocknungsgas arbeiten. Angesprochen ist hier insbesondere die Stofftrocknung mit überhitztem Wasserdampf im Sinne der Offenbarung der DE-A 40 30 688 sowie der weiterführenden Veröffentlichungen gemäß DE-A 42 04 035; 42 04 090; 42 06 050; 42 06 521; 42 06 495; 42 08 773; 42 09 432 und 42 34 376, auf die hier ausdrücklich Bezug genommen wird.

Auch das erfindungsgemäße Verfahren sieht dementsprechend vor, sowohl ein im wesentlichen wasserfreies Einsatzgut der Reinigung durch die Behandlung mit dem SHCF als auch ein wasserhaltiges Einsatzgut dieser Behandlung zu unterwerfen. Hierbei kann das wasserhaltige Einsatzgut insbesondere auch einer wenigstens anteiligen Trocknung zugeführt werden. .

Es leuchtet sofort ein, daß eine Vielzahl spezieller Aussführungsformen hier der erfindungsgemäßen Lehre zu subsummieren ist. Diese Vielgestaltigkeit ergibt sich einerseits aus der Beschaffenheit des Einsatzmaterials, hier insbesondere bezüglich seines Wassergehaltes und der physikalischen Beschaffenheit des wasserfreien Wertstoffes beziehungsweise Wertstoffgemisches unter den Arbeitsbedingungen und unter Normalbedingungen. Zum anderen leitet sich die Vielgestaltigkeit möglicher spezieller Ausführungsformen von der Zielsetzung im Sinne des erfindungsgemäßen Handelns ab: Die erfindungsgemäße Lehre will nicht nur eine klassische Stofftrennung, auch die Trocknung des Wertstoffs beziehungsweise des Wertstoffgemisches kann im Rahmen des Verfahrens inbegriffen sein. Die im Einzelfall einzusetzenden Vorrichtungen beziehungsweise Arbeitsmaßnahmen werden somit weitgehend durch die jeweils gegebenen Sachzusammenhänge und das gesetzte Arbeitsziel bestimmt. Im nachfolgenden werden ohne Anspruch auf Vollständigkeit einzelne charakteristische Beispiele erläutert:

In einer ersten speziellen Ausführungsform kann ein unter Arbeitsbedingungen wasserhaltiges Einsatzgut der erfindungsgemäßen Behandlung unterworfen werden, das unter Verfahrensbedingungen getrocknet und gleichzeitig von nicht wäßrigen, insbesondere dampfflüchtigen Begleitstoffen befreit werden soll. Wie angegeben ist schon die Entfernung des Wassers aus einem wasserhaltigen Wertstoff oder Wertstoffgemisch als destillative Trennung im Sinne des erfindungsgemäßen Handelns zu verstehen, so daß hier die einfache Beschleunigung der Trocknung durch Verwendung des überhitzten SHCF-Stromes eine Verwirklichung der erfindungsgemäßen Lehre bedeuten kann. Die Besonderheiten der Praxis machen das allerdings zu einem kaum auftretenden Sonderfall. Wertstoffe und Wertstoffgemische des großtechnisch praktischen Handelns sind wohl immer wenigstens mit Spuren von Verunreinigungen beladen, die mit der überhitzten SHCF-Phase ausgetragen werden. So können Wertstoffe beziehungsweise Wertstoffgemische in Form wäßriger Slurries im Sinne der Lehre der DE-A 42 37 934 - jetzt aber mit der SHCF-Phase - aufgearbeitet und dabei einerseits getrocknet und andererseits deodorisiert werden.

Die Lehre der Erfindung umfaßt damit unter anderem ein Verfahren das dadurch gekennzeichnet ist, daß feste Wertstoffe oder Wertstoffgemische in fließfähiger und versprühbarer wäßriger Zubereitung zum Einsatz kommen, die unter den Arbeitsbedingungen der Behandlung mit der überhitzten SHCF-Phase zur Ausbildung von Feststoffkörpern mit insbesondere offenporiger Innenstruktur geeignet sind, deren Plastizität und Oberflächenklebrigkeit vorzugsweise aber derart eingeschränkt sind, daß substantielle Verklebungen der Teilchen miteinander und/oder deren offenporigen Innenstruktur auch unter den Bedingungen der Einwirkung des SHCF-Stromes ausscheiden. Insbesondere in diesem Zusammenhang kann es wichtig sein, daß die Wertstoffe oder Wertstoffgemische unter Mitverwendung von wasserlöslichen und/oder feinteiligen wasserunlöslichen anorganischen und/oder organischen Hilfsstoffen verarbeitet werden, die bevorzugt im Trockenzustand fest und nicht klebrig sind.

Die erfindungsgemäße Lehre erfaßt aber auch die Verarbeitung solcher Wertstoffe beziehungsweise Wertstoffgemische, die wasserhaltige Zubereitungsformen von unter Normalbedingungen fließfähigen Wertstoffen und Wertstoffgemischen sind. Als Beispiel für Materialien dieser Art seien wasserhaltige und unter Normalbedingungen fließfähige Alkoxylierungsprodukte von Einsatzverbindungen mit wenigstens einer reaktiven Hydroxylgruppe genannt. Ein Beispiel hierfür sind entsprechende wasserhaltige Zubereitungen niotensidischer Komponenten. Für die meisten Anwendungszwecke solcher Alkoxylate ist die praktisch vollständige Entfernung der im Verfahrensprodukt vorliegenden Restmengen an beispielsweise Ethylenoxid (EO) und/oder Propylenoxid (PO) sowie der als Nebenprodukte bei der Alkoxylierung entstehenden unerwünschten cyclischen Ether, beispielsweise Dioxan, zwingend. Die Erfindung ermöglicht auch hier in einem integralen Verfahrensschritt die wirkungsvolle Abtrennung dieser unerwünschten Spurenverunreinigungen und die gleichzeitige Auftrocknung des ursprünglich wasserhaltigen Gutes im vorbestimmten Ausmaß.

Die erfindungsgemäße Lehre umfaßt die Durchführung eines Trennverfahrens der beschriebenen Art in einstufiger aber auch in mehrstufiger Verfahrensausführung. Dabei können in einer solchen mehrstufigen Arbeitsweise die einzelnen Verfahrensstufen nun zunächst einmal in ihrer technischen Funktion gleich aber auch unterschiedlich ausgelegt sein. So kann beispielsweise ein mehrstufiges Trennverfahren mit 2, 3 oder mehr aufeinanderfolgenden Trennstufen arbeiten, in denen das zu reinigende Einsatzgut unter den jeweils gewählten Arbeitsbedingungen als Flüssigphase vorliegt und in die Trennstufe versprüht wird.

In einer wichtigen Ausführungsform sieht die Lehre der Erfindung die Kombination der hier beschriebenen SHCF-Phasen auf Basis niederer, monofunktioneller Alkohole - beziehungsweise ihre Anwendung in wenigstens einer Trennstufe - mit wenigstens einer weiteren Trennstufe vor, in der von dem Arbeitsprinzip Gebrauch gemacht wird, das insbesondere in der eingangs genannten älteren deutschen Patentanmeldung DE-A-43 07 115 beschrieben ist und sich dadurch kennzeichnet, daß hier bei Arbeitsdruck überhitzter Wasserdampf als alkoholfreies Trennmedium eingesetzt wird. Insbesondere für diese Ausführungsform der Erfindung wird hiermit noch einmal ausdrücklich auf die Offenbarung dieser genannten älteren Anmeldung verwiesen und ihre Ausführungen zum Gegenstand auch der vorliegenden Erfindungsoffenbarung gemacht.

Eine solche Kombination von Trennstufen, die einerseits mit einer überhitzten Dampfphase auf Basis eines alkoholhaltigen Mediums und andererseits auf Basis einer Trenngasphase aus reinem überhitztem Wasserdampf arbeiten, kann in Sonderfällen wichtige Vorteile mit sich bringen. Dabei kann nach den jeweiligen Bedürfnissen die mit reinem überhitztem Wasserdampf arbeitende Trennstufe der mit SHCF auf Alkohol-Basis arbeitenden Trennstufe vor- und/oder nachgeschaltet sein. Damit verbundene Vorteile werden an den folgenden Beispielen verständlich: Wird in der ersten Trennstufe im Sinne des erfindungsgemäßen Handelns mit einer alkoholhaltigen SHCF-Phase gearbeitet, dann fällt ein gereinigtes Verfahrensprodukt an, das - nach Kondensation des dampfförmigen Restalkohols im Verfahrensprodukt - mit geringen Restmengen an Alkohol belastet sein kann. Häufig ist das zu tolerieren. Ist das nicht erwünscht, wird durch eine nachgeschaltete, mit reinem Wasserdampf arbeitende Trennstufe dieser Restalkoholgehalt ausgetragen. Eine Umkehr in der Reihenfolge der Trennstufen bietet sich dann an, wenn im gereinigten Fertigprodukt keine Restmengen an kondensierter Feuchte erwünscht sind, entsprechende Restmengen eines niederen Alkanols aber nicht stören.

Nachfolgend wird die erfindungsgemäße Lehre anhand der beigefügten Figuren 1 bis 3 näher erläutert, die jeweils konkrete Ausgestaltungen des erfindungsgemäßen Arbeitssprinzips darstellen. Diese Ausführungsformen sind lediglich beispielhaft zu verstehen. Einschlägiges Fachwissen ermöglicht anhand dieser Darstellungen vielgestaltige Variationen unter Verwirklichung der die erfindungsgemäße Lehre kennzeichnenden Prinzipien.

Gemäß Figur 1 ist im Kopfteil des Kessels 1 eine Sprühvorrichtung 2 vorgesehen, die durch eine oder eine Mehrzahl von Sprühdüsen - gegebenenfalls auch Mehrstoffsprühdüsen - ausgebildet ist. Das zu behandelnde Einsatzmaterial wird mittels der Pumpe 3 über die Leitungen 4 und 5 dieser Sprühvorrichtung 2 zugeführt. Dabei wird mittels des Wärmetauschers 6 und der Heizvorrichtung 7 die Eintrittstemperatur des zu behandelnden Einsatzmaterials auf die gewünschten Werte eingestellt. Die als dampfförmiges Schleppmittel eingesetzte überhitzte Gasphase wird mittels Pumpe 8 und Leitung 9 dem im unteren Bereich des Kessels 1 angeordneten Verteilerelement 10 zugeführt und strömt innerhalb dieses Kessels im Gegenstrom zum versprühten Einsatzgut. Die gewünschte Temperatur dieser SHCF-Phase im Sinne der erfindungsgemäßen Definition wird mittels des bevorzugt indirekten Heizelementes 11 geregelt. Über die als Alternative vorgesehene Leitung 12 kann wenigstens ein Anteil der überhitzten Dampfphase auch der Sprühvorrichtung 2 im Kopfteil des Kessels 1 zugeführt werden und hier insbesondere als Treibgas beim Sprühvorgang Verwendung finden. Das sich am Boden des Behandlungsraumes ansammelnde Flüssigprodukt 13 wird mittels der Pumpe 14 über Leitung 15 ausgetragen. Als Alternative ist die Kreislaufführung wenigstens eines Anteiles des behandelten Gutes und Rückführung in den Kopf des Behandlungsraumes über die Leitungen 16 und 5 vorgesehen. Hierdurch wird die absatzweise oder auch kontinuierlich geführte Mehrfachversprühung des zu reinigenden Einsatzmaterials mit der überhitzten SHCF-Phase ermöglicht. Die mit den aufgenommenen Verunreinigungen beladene SHCF-Phase wird aus dem Kessel 1 über Leitung 17 mittels des Gebläses 18 abgezogen, der überhitzte Dampfstrom passiert dabei den Wärmetauscher 19. Der hier verflüssigte Anteil des abgezogenen Dampfstromes kann über 20 entnommen werden.

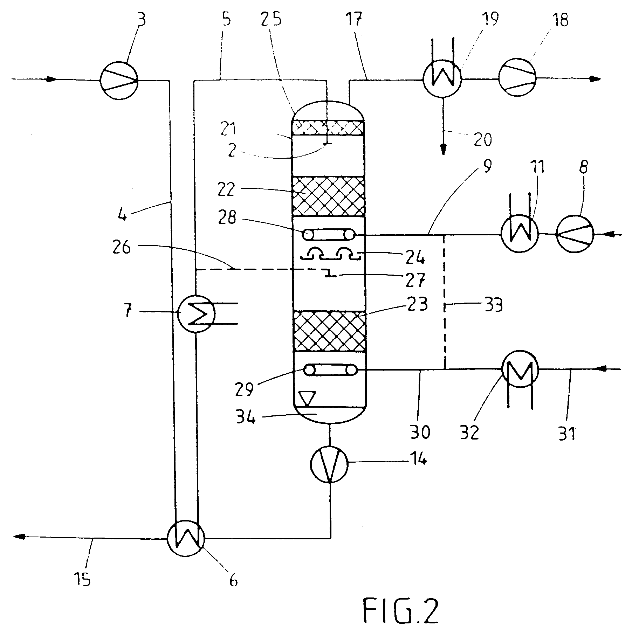

Figur 2 zeigt beispielhaft die Möglichkeit der mehrstufigen Arbeitsweise, wobei konkret hier 2 Arbeitsstufen vorgesehen sind. Die Kolonne 21 ist durch das dampfdurchlässige Trennelement 24, das in der zeichnerischen Darstellung als Glockenboden ausgeführt ist, in 2 Abteilungen unterteilt. Im jeweils unteren Bereich dieser beiden Kolonnenabschnitte sind die Eintragsvorrichtungen 28 und 29 für die überhitzte SHCF-Dampfphase vorgesehen, im Kopfteil des oberen Kolonnenabschnittes wird das zu behandelnde flüssige Einsatzmaterial wieder über eine Sprühvorrichtung 2 eingeführt. Als mögliche Ausgestaltung ist ein entsprechender Eintrag des zu reinigenden Einsatzmaterials auch im Kopfteil des unteren Kolonnenabschnittes über die Sprühvorrichtung 27 vorgesehen. Als zusätzliche Hilfe zur Phasentrennung sind in beiden Kolonnenabschnitten die Packungselemente 22 beziehungsweise 23 vorgesehen, wie sie beispielweise in der heutigen Technologie der Trennkolonnen für Destillation und Absorption bekannt und im Einsatz sind. Entsprechende Packungselemente aus Metall und/oder aus Kunststoff sind insbesondere für die Stofftrennung durch Destillation, Absorption und Desorption in Trennkolonnen gebräuchliche Arbeitselemente, vergleiche beispielsweise den Handelsprospekt "Trennkolonnen für Destillation und Absorption" der Firma Gebrüder Sulzer AG, Produktbereich Chemtech Trennkolonnen, Winterthur, CH (22.13.06.20 - V. 91/100). Im Kopfteil des oberen Kolonnenabschnittes ist oberhalb der Sprühdüsen 2 eine De-Mister-Vorrichtung 25 angeordnet, die beispielsweise als entsprechend dampfdurchlässige Packung ausgebildet ist.

Das als Flüssigphase vorliegende und der Reinigung zu unterwerfende Einsatzmaterial wird mittels der Pumpe 3 über die Leitungen 4 und 5 den Sprühvorrichtungen 2 des oberen Kolonnenteils zugeführt, gewünschtenfalls wird ein Anteil dieses Gutes über Leitung 26 dem Kopfteil des unteren Kolonnenabschnittes und den dort vorgesehenen Sprühdüsen zugeleitet. Auch hier ist - wie in Figur 1 - der Wärmetauscher 6 und zur endgültigen Regulierung der Temperatur des Einsatzmaterials die insbesondere indirekt arbeitende Heizvorrichtung 7 vorgesehen.

Die überhitzte SHCF-Dampfphase wird dem oberen Kolonnenabschnitt mittels Pumpe 8 - nach Durchlauf des Erhitzers 11 - über Leitung 9 durch die Verteilungsvorrichtung 28 zugeführt. Entsprechend ist im unteren Kolonnenabschnitt vorgesehen, eine überhitzte SHCF-Trägerphase über Leitung 31, Heizvorrichtung 32 und Leitung 30 in die Verteilervorrichtung 29 einzuführen. Alternativ besteht die Möglichkeit über Leitung 33 einen Anteil dieses überhitzten Schleppmittels auch unmittelbar in den Bodenteil des oberen Kolonnenabschnittes einzuführen.

Das sich am Boden der Kolonne 21 ansammelnde mehrstufig gereinigte Produkt 34 wird mit Pumpe 14 über Leitung 15 ausgetragen. Das gasförmige und mit den aufgenommenen Stoffanteilen beladene SHCF-Schleppmittel wird am Kopf der Kolonne über Leitung 17 mittels des Gebläses 18 abgezogen. Es passiert den Wärmeaustauscher 19. Der sich hier verflüssigende Anteil kann über 20 entnommen werden.

Die in dieser Figur 2 dargestellte Arbeitsweise ist nicht nur ein Beispiel für die mehrstufige Behandlung im Sinne des erfindungsgemäßen Handelns, insbesondere wird hier auch die Möglichkeit deutlich, wenigstens eine Arbeitsstufe mit der Alkohol enthaltenden SHCF-Phase im Sinne der erfindungsgemäßen Lehre mit einer weiteren Arbeitsstufe zu verbinden, in der überhitzter Wasserdampf als Schleppmittel eingesetzt wird. In der Vorrichtung der Figur 2 läßt sich das beispielsweise dergestalt einfach verwirklichen, daß das zu behandelnde Einsatzmaterial ausschließlich am Kopf der Kolonne durch die Sprühvorrichtung 2 aufgegeben wird. Das zu reinigende Gut passiert dann zunächst die obere Reinigungsstufe mit der Packung 22, durchtritt die Trennebene 24 und passiert nachfolgend die untere Arbeitstufe mit der Packung 23. Im oberen Kolonnenbereich wird über Leitung 9 und Verteilervorrichtung 28 jetzt beispielsweise die alkoholhaltige überhitzte SHCF-Phase im Sinne des Kernes der erfindungsgemäßen Lehre gegeben, während im unteren Kolonnenbereich über Leitung 30 und Verteilervorrichtung 29 überhitzter Wasserdampf als Schleppmittel eingebracht werden kann. Auf diese Weise kann sichergestellt werdene, daß das sich im Boden der Trennkolonne ansammelnde gereinigte Gut 34 von Alkoholrestanteilen befreit ist.

Die erfindungsgemäße Lehre erfaßt in einer bevorzugten Ausführungsform Arbeitsweisen, bei denen die beladene SHCF-Phase nach ihrer Abtrennung von dem gereinigten Gut wenigstens anteilsweise von ihren aus dem Einsatzmaterial aufgenommenen Anteilen befreit und einer weiteren Verwertung zugeführt wird. Dabei ist die wenigstens anteilsweise Rückführung der so gereinigten SHCF-Phase in die Trennstufe(n) eine weiterhin bevorzugte Ausführungsform für viele Anwendungsfälle.

Diese Aufarbeitung der beladenen SHCF-Trennphase kann grundsätzlich in beliebiger, dem Fachmann an sich bekannter Weise vorgenommen werden. Insoweit kann auf das allgemeine Fachwissen verwiesen werden. Besonders wirkungsvoll kann für diesen sekundären Arbeitsschritt im Sinne des erfindungsgemäßen Handelns der Einsatz der Membrantechnologie sein. Zum allgemeinen Fachwissen wird beispielsweise verwiesen auf die in Buchform erschienenen Veröffentlichungen JU.I. Dytnerskij "Membranprozesse zur Trennung flüssiger Gemische" VEB Deutscher Verlag für Grundstoffindustrie, Leipzig 1977 und M. Cheryan "ULTRAFILTRATION HANDBOOK", Technomic Publishing Co, Inc., Lancaster, Basel, 1986.

Die Auswahl und Anpassung der jeweiligen Membrantrennverfahren nach Typ und Beschaffenheit der gewählten Membranen und der jeweils im speziellen einzusetzenden Technologie wird - unter Berücksichtigung des allgemeinen Fachwissens - durch das jeweils zu trennende Stoffgemisch bestimmt. Möglich ist dabei sowohl die Durchführung dieser Membrantrennverfahren unter Ausbildung flüssiger Produktströme als auch der Einsatz der Pervaporation, bei der bekanntlich die die Membran durchtretende Stoffphase als Dampf- beziehungsweise in der Gasphase anfällt. Die Auswahl der jeweils geeigneten Membrantypen reicht von der Mikrofiltration über die Ultra- und Nanofiltration bis zur Umkehrosmose. Auch hier wird im einzelnen das technische Handeln durch die jeweils zu berücksichtigenden Gegebenheiten des aufzutrennenden Stoffgemisches bestimmt.

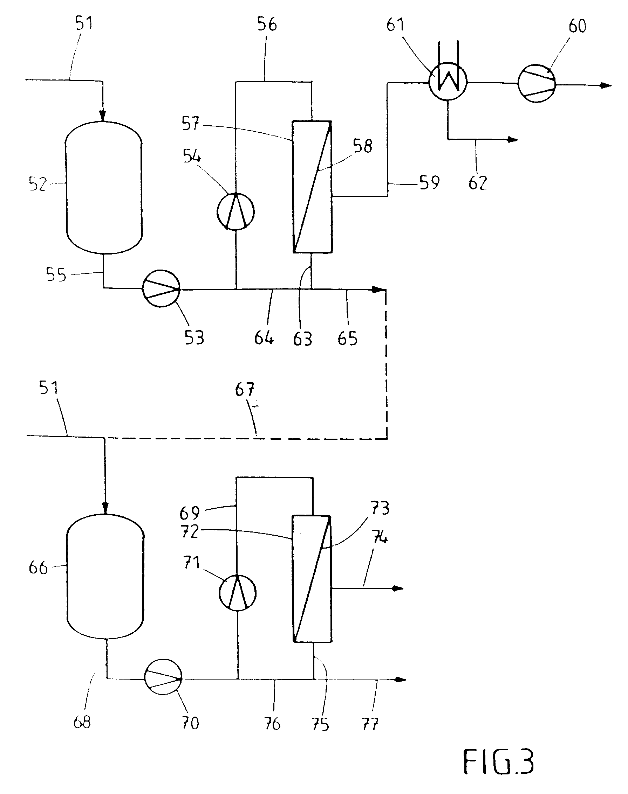

Die Figur 3 zeigt in schematischer Darstellung Möglichkeiten zu einer solchen Stofftrennung an beladenen SHCF-Phasen unter Einsatz der Membrantechnologie.

Das im vorgängigen Reinigungs- beziehungsweise Trennschritt mit Verunreinigungen beladene Trennhilfsmittel auf Basis der niederen monofunktionellen Alkohole oder deren Abmischungen mit Wasser wird als Kondensat über Leitung 51 in den Vorlagebehälter 52 gegeben und mittels der Pumpen 53 und 54 über Leitungen 55 und 56 daraus abgezogen und in die Membrantrennvorrichtung 57 eingespeist. Unter dem eingestellten Arbeitsdruck tritt das Permeat durch die semipermeable Membran 58 und wird mittels des Gebläses 60 über Leitung 59 aus der Membrantrennanlage 57 abgezogen. Der abgezogene Permeatstrom kann in dem Kondensator 61 wenigstens weitgehend kondensiert werden, so daß über Leitung 62 ein gereinigter Produktstrom abgezogen und der Weiterverwertung zugeführt werden kann. Das in der Membrantrennanlage zurückgehaltene Retentat wird über Leitung 63 abgezogen und kann wenigstens anteilsweise über Leitungen 64 und 56 in die Trennstufe zurückgeführt werden. Die Entnahme eines hinreichend aufkonzentrierten Retentats ist über Leitung 65 möglich.

Eine leicht modifizierte Ausführungsform für dieses Trennverfahren, das auch mit der bisher geschilderten Führung von Produktströmen gemäß Figur 3 verbunden werden kann, schließt sich an. Hier wird im Vorlagebehälter 66 über Leitung 51 mit aufgenommenen Verunreinigungen beladenes SHCF als Flüssigphase zugeführt. Alternativ oder zusätzlich kann das aus der Membrantrennanlage 57 abgezogene Retentat 65 über Leitung 67 ebenfalls in den Vortagebehälter 66 gegeben werden. Mittels Pumpen 70 und 71 wird über die Leitungen 68 und 69 das verflüssigte Stoffgemisch der Membrantrennvorrichtung 72 mit der darin angeordneten semipermeablen Membran 73 zugeführt. Gereinigtes Permeat wird über Leitung 74 entnommen. Retentat verläßt über 75 die Membrantrennanlage und kann wenigstens anteilsweise über 76 und 69 im Kreislauf geführt werden. Wenigstens anteilsweise wird aber über Leitung 77 angereichertes Retentat abgezogen und entweder einer Wiederverwertung oder einer angemessenen Entsorgung zugeführt.

Auf einzelne besonders wichtige Anwendungsgebiete der neuen Technologie sei im folgenden noch näher eingegangen:

Besonders interessant wird der Einsatz des erfindungsgemäßen Reinigungsverfahrens immer dann sein, wenn sich diese Reinigung mit niederen Alkoholen als SHCF-Phase einfach in einen größeren Sachzusammenhang integrieren läßt. In der Regel ist das dann der Fall, wenn beispielsweise ein Estergruppen, insbesondere Alkanolester, enthaltendes Einsatzmaterial in einem Verfahrensschritt anfällt und einer nachfolgenden Reinigung unterworfen werden soll. So sind beispielsweise Ester von Fettsäuren oder Fettsäuregemischen mit Alkanolen, insbesondere mit Methanol, Ethanol und/oder (Iso)Propanol großtechnisch in breitem Umfang anfallende Produkte, die in aller Regel einem Reinigungsschritt unterworfen werden müssen. Lediglich beispielhaft seien hier die entsprechenden Ester aus naturstoffbasierten Fettsäuren und Fettsäuregemischen mit niederen monofunktionellen Alkanolen, insbesondere Methanol und/oder Ethanol benannt. Wertstoffgemische dieser Art fallen beispielsweise in großem Umfang bei der Umesterung von Fettsäuretriglyceriden, insbesondere natürlichen Ursprungs, mit den monofunktionellen Alkoholen an. Das Primärprodukt der Umesterung enthält Glycerin und bedarf üblicherweise der Reinigung, die heute insbesondere destillativ erfolgt.

Im Rahmen der erfindungsgemäßen Lehre wird die Reinigung des rohen Umesterungsproduktes und insbesondere die Abtrennung von Glycerin durch einen gegebenenfalls mehrstufig geführten Schritt im Sinne des erfindungsgemäßen Handelns möglich. Dabei steht das als SHCF-Phase einzusetzende Hilfsmittel - beispielsweise Methanol oder Ethanol - im Rahmen des Umesterungssverfahrens ohnehin in hinreichender Menge zur Verfügung. Die erfindungsgemäße Lehre sieht vor, in den Verfahrensablauf den Reinigungsschritt so zu integrieren, daß der zusätzliche Aufwand an Energie und Hilfschemikalien auf ein Minimum reduziert werden kann. Aber auch die Reinigung von Estern aus Fettsäuren und den längerkettigen Fettalkoholen ist ein für die Lehre der Erfindung wichtiges Anwendungsgebiet.

Der Einsatz des erfindungsgemäßen Trennprinzips ist aber nicht auf die hier dargestellten Anwendungsfälle beschränkt. In an sich bekannter Weise kann die Wirtschaftlichkeit des Trennverfahrens schon dadurch sichergestellt werden, daß die beladene und aus der Trennstufe abgezogene SHCF-Phase wenigstens anteilsweise von ihren aus dem Einsatzmaterial aufgenommenen Anteilen befreit und vorzugsweise dann wiederum wenigstens anteilsweise in die Trennstufe zurückgeführt wird.

Auf ein besonders interessantes Arbeitsgebiet sei hier noch eingegangen: Seit vielen Jahren wird in der Fachwelt die Verwendung von Verbrennungskraftstoffen auf Basis von Naturölen und -fetten diskutiert. Besondere Bedeutung kommt dabei nach heutigem Wissen den Methylestern wenigstens überwiegend ungesättigter Fettsäuren beziehungsweise Fettsäuregemischen zu, die unter dem Fachbegriff "Biodiesel" seit Jahren in der Literatur erwähnt werden. Der praktische Einsatz hat sich bis heute nicht wirklich durchsetzen können. Anlaß hierfür sind unter anderem Probleme aus der hinreichenden Reindarstellung der Methylester, die wie alle naturstoffbasierten Chemikalien eine Vielzahl von Mischungskomponenten aufweisen.

Eine besondere Problemstellung kann in diesem Zusammenhang die hinreichende Abtrennung des Glycerins aus dem primären Produkt der Umesterung des Triglycerids mit Methanol sein. Wird das freigesetzte Glycerin nicht wenigstens weitgehend im Fertigprodukt abgereichert, dann treten insbesondere bei niedrigeren Temperaturen im Biodiesel Entmischungen auf, die die Anwendbarkeit dieses Kraftstoffes einschränken.

Die erfindungsgemäße Lehre eröffnet hier eine Möglichkeit unter minimalem zusätzlichem Aufwand an Energie und Hilfschemikalien die Reinigungsstufe mit überhitztem Methanol als SHCF-Phase in den Verfahrensschritt der Gewinnung des Biodiesels zu integrieren und damit die gewünschten Reinstqualiatäten des pflanzlich basierten Kraftstoffes zu verwirklichen.

## Patentansprüche

1. Verfahren zur Trennung eines bei Arbeitstemperaturen fließfähigen, organische Anteile enthaltenden Mehrstoffgemisches (Einsatzmaterial) durch feinteiliges Versprühen des Einsatzmaterials in eine Sprühzone, die von einem gasförmigen Schleppmittel durchströmt wird, das der Sprühzone - bezogen auf deren Arbeitsbedingungen - als überhitzte Dampfphase zugeführt und mit Anteilen des Einsatzmaterials beladen wieder abgetrennt wird, dadurch gekennzeichnet, daß monofunktionelle Alkohole mit 1 bis 3 C-Atomen oder deren Abmischungen mit Wasser als überhitzte Dampfphase und damit als gasförmiges Schleppmittel eingesetzt werden.

2. Verfahren nach Anspruch 1, dadurch gekennzeichnet, daß mit Methanol und/oder Ethanol als Alkoholbasis des gasförmigen Schleppmittels gearbeitet wird.

3. Verfahren nach Ansprüchen 1 und 2, dadurch gekennzeichnet, daß mit Gehalten der niederen monofunktionellen Alkohole im gasförmigen Schleppmittel von wenigstens etwa 10 bis 25 Gew.-%, insbesondere von wenigstens etwa 50 Gew.-% arbeitet, wobei in bevorzugten Ausführungsformen der entsprechende Alkoholgehalt des gasförmigen Schleppmittels wenigstens etwa 80 bis 85 Gew.-% beträgt.

4. Verfahren nach Ansprüchen 1 bis 3, dadurch gekennzeichnet, daß praktisch wasserfreie Alkohole oder deren azeotrope Abmischungen mit Wasser als gasförmiges Schleppmittel eingesetzt werden.

5. Verfahren nach Ansprüchen 1 bis 4, dadurch gekennzeichnet, daß das gasförmige Schleppmittel in die Trennstufe mit Einsatztemperaturen eingebracht wird, die wenigstens 10°C oberhalb des Siedepunktes der höchstsiedenden Komponente des gasförmigen Schleppmittels bei den Arbeitsbedingungen der Trennstufe liegen und vorzugsweise wenigstens 20°C, zweckmäßigerweise wenigstens 50°C und insbesondere wenigstens 100°C jeweils oberhalb des angegebenen Siedepunktes liegen.

6. Verfahren nach Ansprüchen 1 bis 5, dadurch gekennzeichnet, daß das Einsatzmaterial in der Sprühzone mit dem gasförmigen Schleppmittel im Gleich- und/oder Gegenstrom behandelt wird.

7. Verfahren nach Ansprüchen 1 bis 6, dadurch gekennzeichnet, daß das Einsatzmaterial mittels eines Treibgases versprüht wird, wobei gasförmiges Schleppmittel als bevorzugtes Treibgas eingesetzt wird.

8. Verfahren nach Ansprüchen 1 bis 7, dadurch gekennzeichnet, daß das unter den Sprühbedingungen fließfähige Einsatzmaterial mittels Mehrstoffdüsen und/oder Zerstäuberscheiben versprüht wird.

9. Verfahren nach Ansprüchen 1 bis 8, dadurch gekennzeichnet, daß das Einsatzmaterial mit einer Eigentemperatur der Trennstufe zugeführt wird, die wenigstens etwa der Siedetemperatur der höchstsiedenden Komponente des gasförmigen Schleppmittels unter Arbeitsbedingungen entspricht, bevorzugt aber darüber liegt.

10. Verfahren nach Ansprüchen 1 bis 9, dadurch gekennzeichnet, daß man in der Trennstufe - in Abhängigkeit von der Flüchtigkeit des Einsatzmaterials und der zu entfernenden Verunreinigungen - bei Normaldruck oder bei Unterdrucken, gegebenenfalls aber auch bei Überdrucken, arbeitet.

11. Verfahren nach Ansprüchen 1 bis 10, dadurch gekennzeichnet, daß mit aufeinander abgestimmten Temperaturen des Einsatzmaterials und des gasförmigen Schleppmittels gearbeitet wird, wobei wenigstens etwa gleiche Temperaturen, insbesondere aber ein vergleichsweise höheres Temperaturniveau in dem zum Einsatz kommenden gasförmigen Schleppmittel bevorzugt sind.

12. Verfahren nach Ansprüchen 1 bis 11, dadurch gekennzeichnet, daß mit Einsatztemperaturen des gasförmigen Schleppmittels bis 500°C, vorzugsweise im Bereich bis etwa 350°C, gearbeitet wird.

13. Verfahren nach Ansprüchen 1 bis 12, dadurch gekennzeichnet, daß die gasförmige Schleppmittel-Phase in Mengen von 0,5 bis 20 Gew.-%, vorzugsweise 1 bis 15 Gew.-% eingesetzt wird - Gew.-% bezogen auf zu reinigendes Einsatzmaterial.

14. Verfahren nach Ansprüchen 1 bis 13, dadurch gekennzeichnet, daß ein im wesentlichen wasserfreies Einsatzmaterial dem Verfahren unterworfen wird.

15. Verfahren nach Ansprüchen 1 bis 13, dadurch gekennzeichnet, daß ein wasserhaltiges Einsatzmaterial der Behandlung unterworfen und gewünschtenfalls dabei wenigstens anteilig getrocknet wird.

16. Verfahren nach Ansprüchen 1 bis 15, dadurch gekennzeichnet, daß das Trennverfahren mehrstufig durchgeführt wird, wobei in den aufeinanderfolgenden Trennstufen gleiche und/oder von einander verschiedene überhitzte Dampfphasen als gasförmiges Schleppmittel eingesetzt werden können.

17. Verfahren nach Ansprüchen 1 bis 16, dadurch gekennzeichnet, daß in einem mehrstufigen Trennverfahren wenigstens eine Trennstufe mit einer monofunktionellen Alkohole enthaltenden gasförmigen Schleppmittel-Phase und wenigstens eine weitere Arbeitsstufe mit einer alkoholfreien überhitzten Wasserdampfphase als Schleppmittel eingesetzt werden.

18. Verfahren nach Ansprüchen 1 bis 17, dadurch gekennzeichnet, daß eine Estergruppe, insbesondere Alkanol-Ester enhaltendes Einsatzmaterial dem Verfahren unterworfen wird.

19. Verfahren nach Ansprüchen 1 bis 18, dadurch gekennzeichnet, daß Reaktionsgemische aus der Veresterung und/oder Umesterung von Einsatzmaterialien auf Basis von Fettsäuren und Fettsäurederivaten, wie Fettsäureestern, insbesondere aus der Umesterung von Fettsäuretriglyceriden mit monofunktionellen Alkoholen, mittels der niederen Alkanole im überhitzten Zustand gereinigt werden.

20. Verfahren nach Ansprüchen 1 bis 19, dadurch gekennzeichnet, daß die beladene gasförmige Schleppmittel-Phase wenigstens anteilsweise von ihren aus dem Einsatzmaterial aufgenommenen Anteilen befreit und vorzugsweise wiederum wenigstens anteilsweise in die Trennstufe rückgeführt wird.

21. Verfahren nach Ansprüchen 1 bis 20, dadurch gekennzeichnet, daß die Stofftrennung unter Einsatz von Alkanolen als gasförmiges Schleppmittel mit einer Veresterungs- und/oder Umesterungsstufe verbunden wird, in der die Alkanole als Reaktanten und/oder als Reaktionsprodukte vorliegen und das die beladene gasförmige Schleppmittel-Phase - gegebenenfalls nach wenigstens anteilsweiser Abtrennung aufgenommener Anteile des Einsatzmaterials - in diese Arbeitsstufe überführt wird.

22. Anwendung des Verfahrens nach Ansprüchen 1 bis 21 zur Verbesserung des Reinheitsgrades von Wertstoffen und Wertstoffgemischen pflanzlichen und/oder synthetischen Ursprungs, insbesondere von Fetten und/oder Ölen, z.B. für deren Einsatz als Nahrungsmittel, von Komponenten aus dem Bereich der Kosmetika, der Netz-, Wasch- und/oder Reinigungsmittel sowie der pharmazeutischen Hilfsstoffe.

23. Anwendung des Verfahrens nach Ansprüchen 1 bis 21 zur Reinigung der Reaktionsprodukte aus der Umesterung von Glyceridestern, insbesondere Triglyceriden natürlichen Ursprungs, mit monofunktionellen Alkanolen mit insbesondere 1 bis 5 C-Atomen.

24. Anwendung nach Anspruch 23, dadurch gekennzeichnet, daß Fettsäure-Methylester aus der Glycerid-Umesterung mit überhitztem Methanol gereinigt werden.

25. Anwendung des Verfahrens nach Ansprüchen 1 bis 21 zur Reinigung von Verbrennungskraftstoffen auf Basis Fettsäure-Methylester (Biodiesel), insbesondere zur erleichterten Abtrennung freien Glycerins aus dem Produkt der Umesterung mit Methanol.

## Claims

1. A process for the separation of a multicomponent mixture flowable at working temperatures and containing organic components (starting material) by fine-droplet spraying thereof into a spraying zone through which flows a gaseous entraining agent that is delivered to the spraying zone - based on its operating conditions - as a superheated vapour phase and removed again laden with constituents of the starting material, characterized in that lower monohydric alcohols containing 1 to 3 carbon atoms or mixtures thereof with water are used as the superheated vapour phase and hence as the gaseous entraining agent.

2. A process as claimed in claim 1, characterized in that it is carried out with methanol and/or ethanol as the alcohol base of the gaseous entraining agent.

3. A process as claimed in claims 1 and 2, characterized in that it is carried out with contents of the lower monohydric alcohols in the gaseous entraining agent of at least about 10 to 25% by weight and, more particularly, at least about 50% by weight, the corresponding alcohol content of the gaseous entraining agent in preferred embodiments being at least about 80 to 85% by weight.

4. A process as claimed in claims 1 to 3, characterized in that substantially water-free, lower monohydric alcohols or azeotropic mixtures thereof with water are used as the gaseous entraining agent.

5. A process as claimed in claims 1 to 4, characterized in that the gaseous entraining agent is introduced into the separation stage at temperatures which are at least 10°C above the boiling point of the highest-boiling component of the gaseous entraining agent under the working conditions of the separation stage and preferably at least 20°C, more preferably at least 50°C and most preferably at least 100°C above that boiling point.

6. A process as claimed in claims 1 to 5, characterized in that the starting material is treated with the gaseous entraining agent in co-current and/or countercurrent in the spraying zone.

7. A process as claimed in claims 1 to 6, characterized in that the starting material is sprayed by means of a propellent gas, gaseous entraining agent preferably being used as the propellent gas.

8. A process as claimed in claims 1 to 7, characterized in that the starting material flowable under the spraying conditions is sprayed by means of multicomponent nozzles and/or atomizing disks.

9. A process as claimed in claims 1 to 8, characterized in that the starting material is delivered to the separation stage at a temperature which at least substantially corresponds to the boiling temperature of the highest-boiling component of the gaseous entraining agent under working conditions, but is preferably above that temperature.

10. A process as claimed in claims 1 to 9, characterized in that the separation stage is carried out under normal pressure or under reduced pressures and, optionally, even under excess pressures, depending on the volatility of the starting material and the impurities to be removed.

11. A process as claimed in claims 1 to 10, characterized in that the temperatures of the starting material and the gaseous entraining agent are adapted to one another, at least substantially the same temperatures, but especially a higher temperature level in the gaseous entraining agent used being preferred.

12. A process as claimed in claims 1 to 11, characterized in that the gaseous entraining agent is used at temperatures of up to 500°C and preferably at temperatures of up to about 350°C.

13. A process as claimed in claims 1 to 12, characterized in that the gaseous entraining agent phase is used in quantities of 0.5 to 20% by weight and preferably in quantities of 1 to 15% by weight, based on the starting material to be purified.

14. A process as claimed in claims 1 to 13, characterized in that a substantially water-free starting material is subjected to the process.

15. A process as claimed in claims 1 to 13, characterized in that a water-containing starting material is subjected to the treatment and, if desired, is at least partly dried at the same time.

16. A process as claimed in claims 1 to 15, characterized in that the separation process is carried out in several stages, identical and/or different superheated vapor phases being used as the gaseous entraining agent in the successive separation stages.

17. A process as claimed in claims 1 to 16, characterized in that at least one separation stage using a gaseous entraining agent phase containing monohydric alcohols and at least one other working stage using an alcohol-free superheated steam phase as the entraining agent are used in a multistage separation process.

18. A process as claimed in claims 1 to 17, characterized in that a starting material containing ester groups, more especially alkanol esters, is subjected to the process.

19. A process as claimed in claims 1 to 18, characterized in that reaction mixtures from the esterification and/or transesterification of starting materials based on fatty acids and fatty acid derivatives, such as fatty acid esters, more especially from the transesterification of fatty acid triglycerides with monohydric alcohols, are purified with the superheated lower alkanols.

20. A process as claimed in claims 1 to 19, characterized in that the laden gaseous entraining agent phase is at least partly freed from its constituents taken up from the starting material and is preferably returned, again at least partly, to the separation stage.

21. A process as claimed in claims 1 to 20, characterized in that separation using alkanols as the gaseous entraining agent is linked to an esterification and/or transesterification stage in which the alkanols are present as reactants and/or as reaction products and in that the laden gaseous entraining agent phase is transferred to this working stage, optionally after at least partial removal of constituents taken up from the starting material.

22. The use of the process claimed in claims 1 to 21 for improving the purity of useful materials and mixtures of useful materials of vegetable and/or synthetic origin, more especially fats and/or oils, for example for their use as foods, and for improving the purity of components from the field of cosmetics, welling agents, detergents and/or cleaning formulations and also pharmaceutical auxiliaries.

23. The use of the process claimed in claims 1 to 21 for purifying the reaction products from the transesterification of glyceride esters, more especially triglycerides of natural origin, with monohydric alkanols containing in particular 1 to 5 carbon atoms.

24. The use claimed in claim 23, characterized in that fatty acid methyl esters from the transesterification of glycerides are purified with superheated methanol.

25. The use of the process claimed in claims 1 to 21 for the purification of combustion fuels based on fatty acid methyl esters (biodiesel), more especially for the simplified separation of free glycerol from the product of transesterification with methanol.

## Revendications

1. Procédé de séparation d'un mélange de plusieurs substances (matériau d'utilisation) contenant des fractions organiques, fluides aux températures de travail, par pulvérisation finement divisée du matériau d'utilisation dans une zone de pulvérisation, traversé par l'écoulement d'un agent d'entraînement sous forme gazeuse, qui est amené à la zone de pulvérisation - en se référant à ses conditions de travail - sous la forme d'une phase vapeur surchauffée, et qui est de nouveau séparé après avoir été chargée par des fractions du matériau d'utilisation,
caractérisé en ce qu'
on utilise des alcools monofonctionnels comportant de 1 à 3 atomes de C, ou bien leurs mélanges avec de l'eau, à titre de phase vapeur surchauffée et, ainsi, à titre d'agent d'entraînement gazeux.

2. Procédé selon la revendication 1,
caractérisé en ce qu'
on travaille avec du méthanol et/ou de l'éthanol, comme base d'alcool de l'agent d'entraînement gazeux.

3. Procédé selon les revendications 1 et 2,
caractérisé en ce qu'
on travaille avec des teneurs en alcools monofonctionnels inférieurs, dans l'agent d'entraînement gazeux, d'au moins environ 10 à 25 % en poids, en particulier d'au moins environ 50 % en poids, la teneur en alcool de l'agent d'entraînement gazeux étant d'au moins environ 80 à 85 % en poids dans des formes de réalisation préférées.

4. Procédé selon les revendications 1 à 3,
caractérisé en ce qu'
on utilise des alcools pratiquement exempts d'eau et leurs mélanges azéotropes avec de l'eau, comme agent d'entraînement gazeux.

5. Procédé selon les revendications 1 à 4,
caractérisé en ce que
l'agent d'entraînement gazeux est introduit dans l'étage de séparation aux températures d'utilisation, qui sont d'au moins 10°C supérieure au point d'ébullition du composant à point d'ébullition le plus haut de l'agent d'entraînement gazeux pour les conditions de travail de l'étage de séparation et, de préférence, d'au moins 20°C de manière appropriée, d'au moins 50°C et, en particulier, d'au moins 100°C chaque fois au-dessus du point d'ébullition indiqué.

6. Procédé selon les revendications 1 à 5,
caractérisé en ce que
le matériau d'utilisation est traité dans la zone de pulvérisation avec l'agent d'entraînement gazeux, dans un processus à co-courant et/ou à contre-courant.

7. Procédé selon les revendications 1 à 6,
caractérisé en ce que
le matériau d'utilisation est pulvérisé au moyen d'un gaz propulseur, l'agent d'entraînement gazeux étant utilisé sous la forme de gaz propulseur préféré.

8. Procédé selon les revendications 1 à 7,
caractérisé en ce qu'
on pulvérise dans les conditions de pulvérisation le matériau d'utilisation fluide au moyen de buses à plusieurs substances et/ou de disques pulvérisateurs.

9. Procédé selon les revendications 1 à 8,
caractérisé en ce que
le matériau d'utilisation est amené à l'étage de séparation, à une température propre qui correspond au moins à peu près à la température d'ébullition du composant à point d'ébullition le plus élevé de l'agent d'entraînement gazeux dans les conditions de travail, de préférence cependant à une température supérieure à celle-ci.

10. Procédé selon les revendications 1 à 9,
caractérisé en ce qu'
on travaille dans l'étage de séparation - en fonction de la volatilité du matériau d'utilisation et des impuretés à éliminer - à pression normale ou sous des conditions de dépression, le cas échéant également des conditions de surpression.

11. Procédé selon les revendications 1 à 10,
caractérisé en ce qu'
on travaille à des températures adaptées mutuellement du matériau d'utilisation et de l'agent d'entraînement gazeux, au moins des températures à peu près identiques, en particulier cependant un niveau de température relativement plus élevé étant préféré dans l'agent d'entraînement gazeux utilisé.

12. Procédé selon les revendications 1 à 11,
caractérisé en ce qu'
on travaille avec des températures d'utilisation de l'agent d'entraînement gazeux allant jusqu'à 500°C, de préférence dans la plage allant jusqu'à environ 350°C.

13. Procédé selon les revendications 1 à 12,
caractérisé en ce que
les quantités d'agent d'entraînement en phase gazeuse utilisé vont de 0,5 à 20 % en poids, de préférence 1 à 15 % en poids - le pourcentage en poids se référant au matériau d'utilisation à épurer.

14. Procédé selon les revendications 1 à 13,
caractérisé en ce que
le matériau d'utilisation soumis au procédé est pratiquement exempt d'eau.

15. Procédé selon les revendications 1 à 13,
caractérisé en ce que
le matériau d'utilisation soumis au traitement contient de l'eau et, si on le souhaite, est séché au moins partiellement.

16. Procédé selon les revendications 1 à 15,
caractérisé en ce que
le procédé de séparation est conduit en plusieurs étapes, dans les étapes de séparation successives pouvant être utilisées, comme agent d'entraînement gazeux, des phases vapeur surchauffées à des niveaux identiques et/ou différents les uns des autres.

17. Procédé selon les revendications 1 à 16,
caractérisé en ce qu'
on utilise dans un procédé de séparation à plusieurs étapes au moins une étape de séparation ayant une phase d'agent d'entraînement gazeuse, contenant des alcools monofonctionnels, et au moins une autre étape de travail avec une phase de vapeur d'eau surchauffée, exempte d'alcool, comme agent d'entraînement.

18. Procédé selon les revendications 1 à 17,
caractérisé en ce qu'
on soumet au procédé un matériau d'utilisation contenant un groupe ester, en particulier un alcanolester.

19. Procédé selon les revendications 1 à 18,
caractérisé en ce qu'
on épure des mélanges de réaction issus de l'estérification et/ou de la réestérification de matériaux d'utilisation à base d'acides gras et de dérivés d'acides gras, tels que des esters d'acide gras, en particulier issus de la réestérification de triglycérides d'acide gras avec des alcools monofonctionnels, au moyen des alcanols inférieurs, à l'état surchauffé.

20. Procédé selon les revendications 1 à 19,
caractérisé en ce que
la phase d'agent d'entraînement gazeuse chargée est débarrassée au moins en partie de ses fractions situées dans le matériau d'utilisation et, de préférence de nouveau, est recyclée à l'étape de séparation au moins pour une partie.

21. Procédé selon les revendications 1 à 20,
caractérisé en ce que
la séparation de substances avec utilisation d'alcanols comme agents d'entraînement gazeux est liée à une étape d'estérification et/ou de réestérification, dans laquelle les alcanols sont présents à titre de réactants et/ou de produits de réaction, et la phase d'agent d'entraînement gazeux chargée - le cas échéant après au moins avoir effectué une séparation partielle des fractions contenues du matériau d'utilisation - est passée dans cette étape de travail.

22. Application du procédé selon les revendications 1 à 21, pour améliorer le degré de pureté de substances et de mélanges de substances d'origine végétale et/ou synthétique, en particulier de graisses et/ou d'huile, par exemple pour leur utilisation comme agent nutritif, de composants issus du domaine de la cosmétique, des agents de mouillage, lavage et/ou nettoyage, ainsi que des auxiliaires pharmaceutiques.

23. Application du procédé selon les revendications 1 à 21, pour l'épuration des produits de réaction issus de la réestérification des esters de glycérides, en particulier de triglycérides d'origine naturelle, avec des alcanols monofonctionnels, ayant en particulier de 1 à 5 atomes C.

24. Application selon la revendication 23,
caractérisée en ce que
les méthylesters d'acides gras issus de la réestirification des glycérides sont épurés avec du méthanol surchauffé.

25. Application du procédé selon les revendications 1 à 21, pour l'épuration des carburants à base de méthylester d'acide gras (Biodiesel), en particulier pour faciliter la séparation de la glycérine libre à partir du produit de réestérification avec du méthanol.
